# EUROPEAN PATENT SPECIFICATION

(11) **EP 3 017 770 B1**
(45) Date of publication and mention of the grant of the patent: **10.10.2018**
(21) Application number: 13889751.7
(22) Date of filing: 14.08.2013
(51) Int. Cl.: A61B 17/00, A61B 8/12, A61B 8/00, A61B 90/00, A61N 7/00

(54) **SUBROUNDED ULTRASONIC ABLATION CATHETER**
UNTERABGERUNDETER KATHETER ZUR ABLATION MITTELS ULTRASCHALL
CATHÉTER D'ABLATION ULTRASONORE SUBARRONDI

(30) Priority: 19.07.2013 CN 201310306443
(43) Date of publication of application: 11.05.2016
(73) Proprietor: Lepu Medical Technology (Beijing) Co., Ltd., Beijing 102200 (CN)
(72) Inventor: HUANG, Jing, Chongqing 400016 (CN); MA, Changsheng, chongqing 400016 (CN); DONG, Saying, Chongqing 400016 (CN); QIANG, Jun, Chongqing 400016 (CN); WANG, Ting, Chongqing 400016 (CN); XUE, Rui, Chongqing 40006 (CN)
(74) Representative: Nordmeyer, Philipp Werner
(86) International application number: PCT/CN2013/081433
(87) International publication number: WO 2015/007003

(56) References cited:
- EP-A1- 2 311 516
- WO-A2-2012/120495
- CN-A- 1 186 420
- CN-A- 101 073 501
- CN-A- 102 068 308
- CN-U- 203 354 582
- US-A- 5 817 021
- US-A1- 2004 015 065
- US-A1- 2011 257 562
- US-A1- 2012 065 506
- US-A1- 2012 265 227
- US-B1- 6 206 831
- POLITIS M J ET AL: "Tropism in nerve regeneration in vivo. Attraction of regenerating axons by diffusible factors derived from cells in distal nerve stumps of transected peripheral nerves", BRAIN RESEARCH, ELSEVIER, AMSTERDAM, NL, vol. 253, no. 1-2, 16 December 1982 (1982-12-16), pages 1-12, XP024278826, ISSN: 0006-8993, DOI: 10.1016/0006-8993(82)90667-9 [retrieved on 1982-12-16]

## Description

### TECHNICAL FIELD

The present application relates to the technical field of medical devices, and particularly to a sub-rounded ultrasound ablation catheter.

### BACKGROUND

In a catheter-based renal sympathetic denervation treatment, a radiofrequency catheter is used to enter a renal artery in a minimally invasive way and a spiral punctiform ablation is performed in the renal artery. Energy acts on sympathetic nerve fibers on a vascular adventitia and in an adipose tissue via a vascular intima, thereby decreasing a sympathetic tone of a kidney and a body and decreasing a blood-pressure. Currently, the catheter-based renal sympathetic denervation has become a very promising treatment method for a resistant hypertension. In addition, it is discovered from some new researches that decreasing the activities of sympathetic nerves of the kidney and the body not only can reduce the blood-pressure, but also can treat a cardiac failure, help prevent an arrhythmia and a sudden death from occurring, improve a blood glucose metabolism condition, and reduce an inflammation level of the body, etc.. The principle of the radiofrequency catheter performing a kidney sympathetic nerve ablation is to gradually transfer a large amount of heat energy produced by a radiofrequency energy in heat transfer form to the vascular adventitia or tissues around the adventitia via the vascular intima, causing degenerative necrosis of the sympathetic nerves on the vascular adventitia and in the tissues around the vascular adventitia, which may inevitably cause damages to a blood vessel endothelium in an ablation area, and it is found out that some patients have suffered a renal artery stenosis complication in long-term follow-up.

Ultrasound energy has a good directivity and a good tissue penetration. Compared with radiofrequency ablation, the energy consumed by ultrasound ablation is lower. When sound energy is being emitted, a wafer need not to contact with the tissues directly, and the ablation can be realized without breaking the intima, therefore the ultrasound energy used as an energy resource for a renal sympathetic denervation has a high efficiency and a better safety.

Research shows that after a nerve fiber is segmentally injured, a nerve at a far end can produce a neurotrophic and chemotaxis effect to a nerve at a near end, and a tube-like connection is regenerated between broken ends of the nerve. When an interval between the broken ends of the nerve is 5mm, it presents the strongest chemotaxis. In contrast, when the interval between the broken ends of the nerve is relatively large, dispersive ineurotrophy and chemotactic factor cannot reach the effective concentrations, and a research (Politis MJ, et al. Tropism in nerve regeneration in vivo. Attraction of regenerating axons by diffusible factors derived from cells in distal nerve stumps of transected peripheral nerves. Brain Res. 1982;253:1-12.) shows that the self-regeneration function is almost completely lost when the interval between the broken ends of the nerve is greater than 10mm. Therefore, when the ablation is being performed, if a short electrode or wafer is used, the sympathetic nerve fiber may be self-restored after operation, adversely affecting a therapeutic effect in the long-term; if an overlong ablation electrode or wafer is used, the released energy is greatly enhanced, which may increase a risk of the blood vessel being injured and an ablation complication.

Besides, according to researches on the conventional technology, the inventor of the present application has found that: all the conventional catheters used in renal sympathetic denervation cannot identify the distribution condition in a periphery of the renal artery of the renal sympathetic nerves. A spiral ablation or a punctiform ablation is one kind of ablation based on "experience", which has a certain blindness in operation and cannot efficiently perform the ablation to the renal sympathetic nerve fiber, and brings unnecessary damages to tissues where no nerve is distributed. A circumferential ultrasound renal sympathetic denervation ablation is another kind of ablation. The circumferential ultrasound ablation may result in a complete sympathetic denervation effect, and it is known that a sympathetic nerve response is one of the important mechanisms for maintaining body homeostasis. Therefore, if the circumferential ultrasound ablation is employed, an excessive sympathetic denervation may occur, which may decrease a stress ability of the patient after the operation. The body of the patient cannot defense against a destructive stimulus from the outside, for example, the body cannot maintain recycling capacity, electrolyte level and the stability of blood pressure, which may dramatically decrease the stress ability of the body. In case of a blood loss or an electrolyte disturbance, etc., a mortality rate of the patient may even be increased, and, the circumferential ultrasound ablation may aggravate the damage to the intima of the renal arteries, thereby resulting in severe complications such as arterial stenosis, fibrosis and thrombosis after operation.

Therefore, the conventional catheter used in renal sympathetic denervation treatment is unable to meet the clinical demands, and a new ablation catheter is presently needed.

US5817021A discloses a therapy apparatus and method for treating conditions of the heart and, particularly, of heart-proximate vessels with therapeutic ultrasound waves having a therapeutically effective region. The therapy apparatus generates therapeutic ultrasound waves with such an intensity that tissue modifications, particularly necrotization, are produced in the tissue located in the region of influence. The therapy apparatus preferably contains an ultrasound source that can be transesophageally applied.

WO2012120495 A2 discloses an apparatus, which includes at least one ultrasound transducer. The ultrasound transducer is configured to be positioned within a lumen of a subject and to ablate tissue surrounding a wall of the lumen without ablating the wall of the lumen, by focusing ultrasound energy on a focal zone that is outside of the wall of the lumen. A transluminal delivery tool is configured to position the ultrasound transducer in the lumen, and a control unit is configured to drive the ultrasound transducer.

US2012265227A1 discloses a method of treating a subject suffering from a nerve related disorder, the method comprising causing a damage region to one or both of a lumen wall or nearby surrounding tissues, the damage region encompassing a volume having dimensions of less than about 6.8 mm in a substantially radial direction, less than about 5.8 mm in a direction substantially tangential to the lumen, less than about 10 mm in a substantially axial direction, the damage region being located no closer than about 0.2 mm from an inner wall of the lumen, the damage region comprises of greater than about 60% of collagen denatured tissue.

### SUMMARY

In view of this, an ultrasound ablation catheter is provided in embodiments of the present application.

For achieving the above object, a technical solution according to an embodiment of the present application is described as follows.

An ultrasound ablation neuromodulation catheter includes a catheter body, an ultrasound treatment transducer assembly, an ultrasound imaging transducer assembly, a control handle, and a transducer interface, in which
the catheter body is of a cylindrical structure;
the ultrasound treatment transducer assembly is arranged on a lateral surface of a far end of the catheter body, and the ultrasound treatment transducer assembly includes at least two ultrasound treatment transducers, and a distance between two adjacent ultrasound treatment transducers is greater than or equal to 1cm;
the ultrasound imaging transducer assembly includes at least one ultrasound imaging transducer, and the ultrasound imaging transducer is arranged between two adjacent ultrasound treatment transducers;
the control handle is arranged at a near end of the catheter body;
the transducer interface includes a first interface connected to the ultrasound treatment transducer assembly and a second interface connected to the ultrasound imaging transducer,
wherein each of the ultrasound treatment transducers is an unclosed circumferential wafer and a circumference angle of the unclosed circumferential wafer is greater than 0 degree and less than 360 degrees, wherein gaps without emitting acoustic beams of at least two unclosed circumferential transducers, are in parallel with or staggered with each other and an axis of the unclosed circumferential wafer coincides with the axis of the catheter body, and wherein the ultrasound ablation neuromodulation catheter further includes at least one independent ultrasound imaging transducer, which is of a strip shape, wherein the independent ultrasound imaging transducer is located at the gap portion of the unclosed circumferential wafer.

Preferably, the at least two ultrasound treatment transducers are in a same curved surface, and acoustic beams emitted by each of the ultrasound treatment transducers are in parallel with or staggered with each other.

Preferably, each of the ultrasound treatment transducers is a piezo-electric wafer visible under X-ray, and a coating film visible under the X-ray is coated on a surface of the piezo-electric wafer.

Preferably, the ultrasound imaging transducer is the circumferential wafer, and an axis of the circumferential wafer of the ultrasound imaging transducer coincides with an axis of the unclosed circumferential wafer of each of the ultrasound treatment transducers.

Preferably, the control handle is configured to control the catheter body to unidirectionally rotate.

A spring piece and a wedge-shaped gear are provided in the control handle, and the catheter body is rotatably connected to the control handle via the wedge-shaped gear.

The spring piece has one end fixed to the control handle and another end pressing against the wedge-shaped gear for preventing the wedge-shaped gear from reversely rotating.

Preferably, a dial for determining a rotation angle of the catheter body is provided on a circumferential surface of the control handle.

Preferably, the ultrasound ablation catheter further includes at least two developing markers visible under the X-ray.

The developing markers are configured to assist the operator in obtaining a position of the ultrasound ablation catheter entering a human body cavity and the rotation angle of the ultrasound ablation catheter.

At least two developing markers are separately located on the catheter body at positions at two ends of the ultrasound treatment transducers.

Each of the developing markers arranged on the catheter body is sheet-shaped. According to the above technical solutions, in the ultrasound ablation catheter according to the embodiments of the present application, the ultrasound treatment transducers and at least one ultrasound imaging transducer are provided at the portion at the far-end of the catheter body, the ultrasound imaging transducer is located between two adjacent ultrasound treatment transducers, and the distance between two adjacent ultrasound treatment transducers is set to be greater than or equal to 1cm, thus when the ablation treatment is being performed, the ultrasound treatment transducers laterally emit the non-focusing acoustic beams in parallel with or staggered with each other to the catheter body, and the rotation of the catheter body may allow the ultrasound treatment transducers to axially rotate (by an angle of zero degree to any degree) in the blood vessel and release heat. After ablation is performed to a sympathetic nerve fiber, the sympathetic nerve fiber may be cut off into sections disconnected to each other and have a middle section completely disconnected, and a reaching distance of each of the acoustic beams emitted by the ultrasound treatment transducers is relatively larger (≥1cm), which further lead the completely disconnected nerve fiber sections cannot perform the chemotaxis effect with each other and cannot be self-restored, thereby stabilizing an sympathetic denervation.

Besides, when the ablation catheter according to the present application is in use, the unclosed circumferential treatment transducers emit sub-rounded (such as a three-fourths quadrant or two-thirds circumference) ultrasound acoustic beams, to generate an sub-rounded nerve ablation effect. When an effective renal-nerve ablation is reached, a part of sympathetic nerve tracts are controllably preserved to keep a basic sympathetic compensative capacity of the body. The invention is defined by the appended claims.

### BRIEF DESCRIPTION OF THE DRAWINGS

For more clearly illustrating embodiments of the present application or the technical solution in the conventional technology, drawings referred to describe the embodiments or the conventional technology will be briefly described hereinafter. Apparently, the drawings in the following description are only several embodiments of the present invention.
Figure 1 is an overall top schematic view of an ultrasound ablation catheter according to an embodiment of the present application;
Figure 2 is a schematic view showing a partial structure of an ultrasound ablation catheter according to an arrangement not part of the invention;
Figure 3 is a side schematic view of the structure in Figure 2; and
Figure 4 is schematic view showing a partial structure of another ultrasound ablation catheter according to an embodiment of the present application.

### DETAILED DESCRIPTION

For those skilled in the art to better understand technical solutions of the present application, the technical solutions in the embodiments of the present application will be described clearly and completely hereinafter in conjunction with the drawings in the embodiments of the present application. Apparently, the described embodiments are only a part of the embodiments of the present application, rather than all embodiments.

Research shows that after a nerve fiber is segmentally injured, a nerve in a far end can produce a neurotrophic effect and a chemotaxis effect to a nerve in a near end, and a tube-like connection is regenerated between broken ends of the nerve, and when an interval between the broken ends of the nerve is 5mm, it presents the strongest chemotaxis. However, when the interval between the broken ends of the nerve is relatively large, dispersive ineurotrophy and chemotactic factor cannot reach the effective concentrations, and a research (Politis MJ, et al. Tropism in nerve regeneration in vivo. Attraction of regenerating axons by diffusible factors derived from cells in distal nerve stumps of transected peripheral nerves. Brain Res. 1982;253:1-12.) shows that the self-regeneration function is almost completely lost when the interval between the broken ends of the nerve is greater than 10mm. Therefore, when the ablation is being performed, if a short electrode or wafer is used, the sympathetic nerve fiber can be self-restored after operation, thus a therapeutic effect in the long-term is adversely affected; if an overlong ablation electrode or wafer is used, the released energy is greatly enhanced, increasing a risk of the blood vessel is injured and an ablation complication.

Therefore, considering the effective width and range of an ablation electrode, a new ultrasound ablation catheter is provided by the present application.

A first embodiment is described as follows.

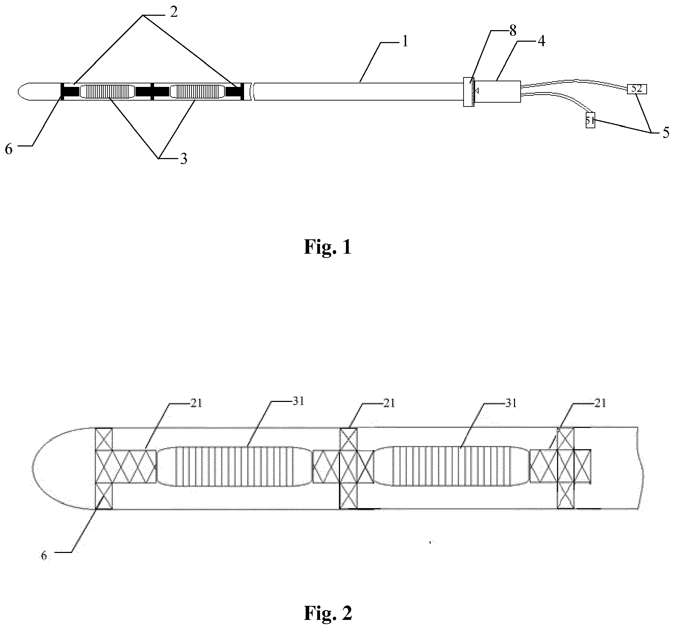

Figure 1 is an overall top schematic view of an ultrasound ablation catheter according to an embodiment of the present application.

As shown in Figure 1, the ultrasound ablation catheter includes a catheter body 1, an ultrasound treatment transducer assembly 2, an ultrasound imaging transducer assembly 3, a control handle 4, and a transducer interface 5.

Both of the ultrasound treatment transducer assembly 2 and the ultrasound imaging transducer assembly 3 are arranged on a lateral surface of a far end of the catheter body 1, and the control handle 4 is arranged at a position at a near end of the catheter body 1. The transducer interface 5 includes a first interface 51 connected to the ultrasound treatment transducer assembly 2 and a second interface 52 connected to the ultrasound imaging transducer assembly 3. The transducer interface 5 is provided for powering the ultrasound treatment transducer assembly 2 and receiving imaging data from the ultrasound imaging transducer assembly 3.

It should be noted that, "far end", "near end" and other position relationships in the present application all refer to a relative position relationship between the ablation catheter and an operator of the ablation catheter, in which "far end" refers to a position away from the operator, and "near end" refers to a position near the operator.

As shown in Figure 1, the catheter body 1 is of a cylindrical structure. A groove is provided on the lateral surface of a far end of the catheter body 1 for accommodating the ultrasound treatment transducer assembly 2 and the ultrasound imaging transducer assembly 3. Moreover, the catheter body 1 may be of a hollow structure for accommodating a cable between the ultrasound treatment transducer assembly 2 and the ultrasound imaging transducer assembly and the transducer interface 7. The catheter body 1 is provided for supporting the whole ultrasound ablation catheter, such that the arranged ultrasound treatment transducer assembly 2 and the ultrasound imaging transducer assembly 3 may be sent into a human body cavity. In addition, a diameter of the catheter body 1 may have multiple options according to clinical requirements.

Figure 2 is a schematic view showing a partial structure of an ultrasound ablation catheter according to an arrangement not part of the invention.

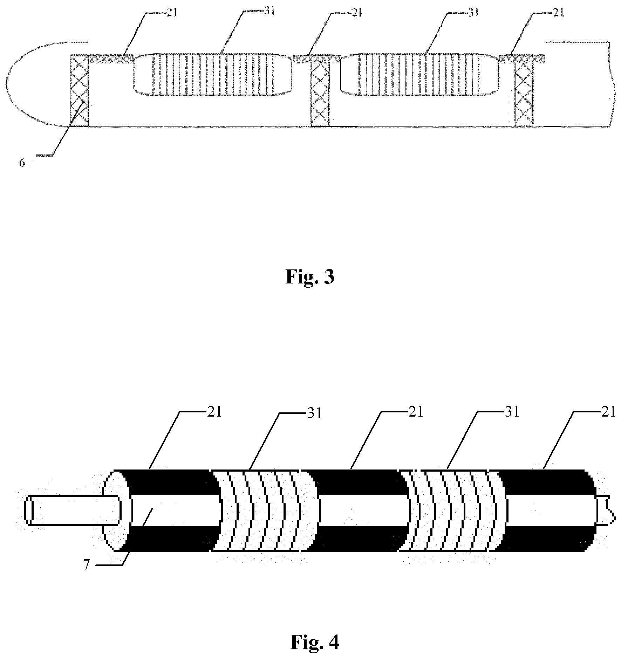

Figure 3 is a side schematic view of the structure in Figure 2.

The ultrasound treatment transducer assembly 2 includes at least two ultrasound treatment transducers 21, which are located at a same plane or a same curved surface, and acoustic beams emitted by the ultrasound treatment transducers 2 are in parallel with each other or staggered with each other, thus the emitted acoustic beams does not focus. The ultrasound treatment transducers 21 are provided for emitting an adjustable ultrasound acoustic beam, so as to ablate nerve fibers on tunica adventitia of artery.

As shown in Figure 2 and Figure 3, each of the ultrasound treatment transducers 21 is a planar wafer, and the at least two ultrasound treatment transducers 21 are in a same plane. Further, long axes of the at least two ultrasound treatment transducers 21 are in a same straight line, and each of the at least two ultrasound treatment transducers 21 is in parallel with an axial direction of the catheter body 1.

In the case that each of the ultrasound treatment transducers 21 is the planar wafer, when the ablation is being performed, the control handle 4is controlled to rotate the catheter body 1 and the at least two ultrasound treatment transducers 21 are further rotated unidirectionally, thereby performing a sub-rounded ablation to nerves in a controllable angle.

The ultrasound treatment transducer assembly 2 includes at least two ultrasound treatment transducers 21. The number of the ultrasound treatment transducers 21 is three as shown in Figures 2 and 3, and a distance between two adjacent ultrasound treatment transducers 21 is greater than or equal to 1cm.

The distance between two adjacent ultrasound treatment transducers 21 is set to be greater than or equal to 1cm. Thus, when the ultrasound ablation catheter is used to perform an ablation treatment, after the two adjacent ultrasound treatment transducers 21 perform the sub-rounded ablation, a distance between two adjacent ablation points that formed is greater than or equal to 1cm. The reason for this is that the self-regeneration function is almost completely lost when the interval between the broken ends of the nerve is greater than 10mm, which may further avoid the sympathetic nerve fiber being self-restored after the operation and adversely affect a long-term treatment effect.

In the embodiment of the present application, each of the ultrasound treatment transducers 21 may be formed by a piezo-electric wafer which is visible under X-ray and a coating film visible under the X-ray, such as gold, platinum, etc., is coated on a surface of the piezo-electric wafer.

As shown in Figure 2 and Figure 3, the ultrasound imaging transducer assembly 3 includes at least one ultrasound imaging transducer 31, which is arranged between two adjacent ultrasound treatment transducers 21. That is to say, the number of the ultrasound imaging transducer 31 is one less than the number of the ultrasound treatment transducers 21. At least one ultrasound imaging transducer 31 is in a same plane or a same curved surface, and each of the ultrasound treatment transducers 21 is in an imaging area of adjacent ultrasound imaging transducer 31. The ultrasound imaging transducer 31 is to provide a convex-array-form high-frequency ultrasound images of tissues at a periphery of an artery, display a distribution condition of the nerve fiber and monitor a difference between images before and after the ablation, and eliminate an interference of tissues at a periphery of a target area to the images by connecting an ultrasound subtraction image analysis system, thereby accurately evaluating an instant effect of the ablation.

As shown in Figure 2 and Figure 3, in the case that each of the ultrasound treatment transducers 21 is the planar wafer, the ultrasound imaging transducer 31 may be a strip-shaped wafer with an arc-shaped surface, and a long axis of each of the at least one ultrasound imaging transducer 31 coincides with the long axis of each of the ultrasound treatment transducers 21, that is, the ultrasound imaging transducer 31 and the ultrasound treatment transducers 21 are in a same straight line.

According to the present invention, addition, as shown in Figure 4, when each of the ultrasound treatment transducers 21 is an unclosed circumferential wafer, the ultrasound imaging transducer 31 may be an circumferential wafer, and an axis of the circumferential wafer of the ultrasound imaging transducer 31 coincides with an axis of the unclosed circumferential wafer of each of the ultrasound treatment transducers 21. That is, the ultrasound imaging transducer 31 and the ultrasound treatment transducers 21 are in a same straight line.

As shown in Figure 1, the control handle 4 is located at a near end of the catheter body 1, and the control handle 4 is provided for unidirectionally rotating the catheter body 1, and then the sub-rounded ablation is performed by the ultrasound treatment transducers 21 arranged in the catheter body 1 in a controllable angle.

In the embodiment of the present application, a spring piece and a wedge-shaped gear are mounted in the control handle 4, and the catheter body 1 is rotatably connected to the control handle via the wedge-shaped gear. In addition, the spring piece has one end fixed to the control handle 4 and another end pressing against the wedge-shaped gear for preventing the wedge-shaped gear from reversely rotating.

For conveniently controlling a rotation angle of the control handle 4, a dial 8 for determining a rotation angle of the catheter body may be provided on a surface of the control handle 4, as shown in Figure 1.

For more conveniently and accurately controlling the rotation angle of the control handle 4, a stepping motor may be installed in the control handle 4, which may allow the wedge-shaped gear to rotate in a pre-set direction by a fixed angle when the stepping motor is connected to a pulse signal source.

Moreover, in order to conveniently determine a position of the ablation catheter entering a human body cavity and to control the rotation angle of the ablation catheter when an ablation operation is being performed. The ablation catheter may be provided with at least two developing markers 6, as shown in Figures 1 to 3.

The at least two developing markers 6 are made of a material visible under the X-ray, the at least two developing markers 6 are separately located at the catheter body 1 and at two ends of the ultrasound treatment transducers 21.

For helping the operator easily obtain the position of the ultrasound ablation catheter in the human body and the rotation angle thereof, in the case that each of the ultrasound treatment transducers 21 is the planar wafer, each of the developing markers 6 is semi-annular, and each of the semi-annular developing markers 6 has two ends fixed to two ends of the respective planar wafer, thus when the catheter body 1 is rotating, the operator may determine the positions of the ultrasound treatment transducers 21 according to the shapes of the developing markers 6.

According to the above technical solutions, in the ablation catheter according to the embodiment of the present application, the ultrasound treatment transducers and at least one ultrasound imaging transducer are provided at the portion at the far-end of the catheter body, the ultrasound imaging transducer is located between two adjacent ultrasound treatment transducers, and the distance between two adjacent ultrasound treatment transducers is set to be greater than or equal to 1cm. Thus when the ablation treatment is being performed, the ultrasound treatment transducers laterally emit the acoustic beams in parallel with or staggered with each other to the catheter body 1 which are not focused. Rotating the catheter body 1 may allow the ultrasound treatment transducers to axially rotate (by an angle of zero degree to any degree) in the blood vessel and release energy. After the ablation is performed to sympathetic nerve fiber, the sympathetic nerve fiber may be cut off into sections disconnected to each other and having a middle section completely disconnected, and a reaching distance (≥ 1cm) of each of the acoustic beams emitted by the ultrasound treatment transducers 21 may further lead the completely disconnected nerve fiber sections cannot perform the chemotaxis effect on each other and be self-restored, thereby stabilizing the sympathetic denervation effect.

When the ablation catheter according to the present invention is in use, the unclosed circumferential treatment transducers emit sub-rounded (such as a three-fourths quadrant or two-thirds circumference) ultrasound acoustic beams, to produce an sub-rounded renal-nerve ablation effect, and while an effective renal-nerve ablation is performed, a part of sympathetic nerve tracts are controllably preserved to keep a basic sympathetic compensative capacity of the body.

The present invention is described as follows.

In the above arrangements, in the case that the ultrasound treatment transducer 21 is the planar wafer, when the ablation is being performed, the control handle 4 is provided for controlling the rotation of the catheter body 1, and the ultrasound treatment transducer 21 is further moved circumferentially, thereby performing a circumferential ablation to the nerves. Therefore, during the operation, an operator is required to monitor the ablation area in real time and rotate the catheter body 1 slowly, which may increase the operating difficulty to the operator and proposes high requirements to the operator. An improper operating which may adversely affect the ablation effect may even occur.

For addressing the issue that the catheter body 1 is required to be rotated to perform the sub-rounded ablation, each of the ultrasound treatment transducers 21 is an unclosed circumferential wafer, as shown in Figure 4. A circumference angle of the unclosed circumferential wafer of each of the ultrasound treatment transducers 21 is greater than 0 degree and less than 360 degrees, and at least two ultrasound treatment transducers 21 are in a same curved plane. Besides, an axis of each of the unclosed circumferential wafers coincides with an axis of the catheter body 1, gaps of the unclosed circumferential wafers are coaxially or uncoaxially in parallel with each other, which makes all of the ultrasound treatment transducers 21 emit acoustic beams in parallel with each other or staggered to cover a distribution range of the renal sympathetic nerves.

The unclosed circumferential wafer is employed, thus when the ablation is being performed, the catheter body 1 is not required to be rotated to perform the sub-rounded ablation to the nerves, thereby allowing the ablation operation to be simply and conveniently operated.

In addition, the ultrasound ablation catheter includes multiple independent ultrasound imaging transducers 7.

Each of the independent ultrasound imaging transducers 7 is located at the gap of the unclosed circumferential wafer of one ultrasound ablation catheter. Each of the independent ultrasound imaging transducers 7 may be of a planar structure, or may form a complete annular shape with the unclosed circumferential wafer.

The independent ultrasound imaging transducers 7 are provided for rotating the catheter body 1 after using the ultrasound imaging transducer 31 to find the ablation area, and the independent ultrasound imaging transducers 7 are used to scan the ablation area to know an overall distribution position of the nerves, etc..

Moreover, each of the developing markers 6 arranged on the catheter body 1 may be sheet-shaped. The sheet-shaped developing markers 6 may be located at the gap of the unclosed circumferential wafer, and a long axis of each of the developing markers 6 coincides with a long axis of each of the unclosed circumferential wafers of the ultrasound treatment transducers 21.

The ultrasound ablation catheter provided by the present application is described in details hereinabove, the principle and the embodiments of the present application are illustrated herein by specific examples, and the above description of examples is only intended to help the understanding of the invention.

The above embodiments and arrangements are described in a progressive manner. Each of the embodiments or arrangements is mainly focused on describing its differences from other embodiments, and references may be made among these with respect to the same or similar portions among these embodiments or arrangements.

It should be noted that, relation description in the present application such as "greater than", "exceed", "higher than", "less than", "lower than" can be interpreted as "greater than or not equal to" or "less than or not equal to"; and can also be interpreted as "greater than or equal to" or "less than or equal to", however should not necessarily be required or implied to be one restrictive or intrinsic condition.

In addition, terms indicating the relationships such as "first" and "second" herein are only used to distinguish an entity or an operation from another entity or operation, and are not intended to demand or indicate that there is any actual relationship or order between the entities or operations. And in the present application, the terms "include", "including" or any other variations thereof are intended to cover a non-exclusive inclusion, such that a process, method, article, or apparatus that comprises a list of elements does not include only those elements but may include other elements not expressly listed or inherent to such process, method, article, or apparatus. Without more constraints, an element proceeded by "comprises a..." does not preclude the existence of additional identical elements in the process, method, article, or apparatus that comprises the element.

The invention is defined by the appended claims.

## Claims

1. An ultrasound ablation neuromodulation catheter, comprising a catheter body (1), an ultrasound treatment transducer assembly (2), an ultrasound imaging transducer assembly (3), a control handle (4), and a transducer interface (5), wherein
the catheter body (1) is of a cylindrical structure;
the ultrasound treatment transducer assembly (2) is arranged on a lateral surface of a far end of the catheter body (1), the ultrasound treatment transducer assembly (2) comprises at least two ultrasound treatment transducers (21), and a distance between two adjacent ultrasound treatment transducers (21) is greater than or equal to 1cm;
the ultrasound imaging transducer assembly (3) comprises at least one ultrasound imaging transducer (31), and the ultrasound imaging transducer (31) is arranged between two adjacent ultrasound treatment transducers (21);
the control handle (4) is arranged at a near end of the catheter body (1); and
the transducer interface (5) comprises a first interface (51) connected to the ultrasound treatment transducer assembly (2) and a second interface (52) connected to the ultrasound imaging transducer (31),
wherein each of the ultrasound treatment transducers (21) is an unclosed circumferential wafer and a circumference angle of the unclosed circumferential wafer is greater than 0 degree and less than 360 degrees,
wherein gaps without transmitting acoustic beams of at least two unclosed circumferential transducers are configured to be in parallel with or staggered with each other,
wherein an axis of each of the unclosed circumferential wafers coincides with the axis of the catheter body (1),
**characterized in that**
the ultrasound ablation neuromodulation catheter further comprises at least one independent ultrasound imaging transducer (7), which is of a strip shape and is located at the gap of the unclosed circumferential wafer.

2. The ultrasound ablation neuromodulation catheter according to claim 1, **characterized in that** each of the ultrasound treatment transducers (21) is a piezo-electric wafer visible under X-ray, and a coating film visible under the X-ray is coated on a surface of the piezo-electric wafer.

3. The ultrasound ablation neuromodulation catheter according to claim 2, **characterized in that** the ultrasound imaging transducer (31) is a strip-shaped wafer with an arc-shaped surface or a circumferential wafer, and the acoustic beams emitted by each of the ultrasound treatment transducers (21) are in an imaging area of the adjacent ultrasound imaging transducer (31).

4. The ultrasound ablation neuromodulation catheter according to claim 3, **characterized in that**
the ultrasound imaging transducer (31) is the circumferential wafer, and the circumferential wafer of the ultrasound imaging transducer (31) has an axis coinciding with an axis of the unclosed circumferential wafer of each of the ultrasound treatment transducers (21).

5. The ultrasound ablation neuromodulation catheter according to claim 4, **characterized in that** the control handle (4) is configured to control the catheter body (1) to unidirectionally rotate;
a spring piece and a wedge-shaped gear are provided in the control handle (4), and the catheter body (1) is rotatably connected to the control handle (4) via the wedge-shaped gear; and
the spring piece has one end fixed to the control handle (4) and another end pressing against the wedge-shaped gear for preventing the wedge-shaped gear from reversely rotating.

6. The ultrasound ablation neuromodulation catheter according to claim 5, **characterized in that** a dial (8) for determining a rotation angle of the catheter body (1) is provided on a circumferential surface of the control handle (4).

7. The ultrasound ablation neuromodulation catheter according to claim 6, **characterized in** further comprising at least two developing markers (6) visible under the X-ray, wherein
the developing markers (6) are configured to assist the operator in obtaining a position of the ultrasound ablation catheter entering a human body cavity and the rotation angle of the ultrasound ablation catheter;
at least two developing markers (6) are separately located at the catheter body (1) at positions at two ends of the ultrasound treatment transducers (21); and
each of the developing markers (6) arranged on the catheter body (1) is sheet-shaped.

## Patentansprüche

1. Ultraschallablations-Neuromodulationskatheter, umfassend einen Katheterkörper (1), eine Ultraschallbehandlungs-Wandler-Baueinheit (2), eine Ultraschallabbildungs-Wandler-Baueinheit (3), einen Steuergriff (4) und eine Wandler-Schnittstelle (5), wobei
der Katheterkörper (1) von einer zylindrischen Struktur ist;
die Ultraschallbehandlungs-Wandler-Baueinheit (2) auf einer seitlichen Oberfläche von einem fernen Ende des Katheterkörpers (1) angeordnet ist, wobei die Ultraschallbehandlungs-Wandler-Baueinheit (2) mindestens zwei Ultraschallbehandlungs-Wandler (21) umfasst, und ein Abstand zwischen zwei benachbarten Ultraschallbehandlungs-Wandlern (21) größer als oder gleich 1 cm ist;
die Ultraschallabbildungs-Wandler-Baueinheit (3) mindestens einen Ultraschallabbildungs-Wandler (31) umfasst, und der Ultraschallabbildungs-Wandler (31) zwischen zwei benachbarten Ultraschallbehandlungs-Wandlern (21) angeordnet ist;
der Steuergriff (4) an einem nahen Ende des Katheterkörpers (1) angeordnet ist; und
die Wandler-Schnittstelle (5) eine erste Schnittstelle (51), verbunden mit der Ultraschallbehandlungs-Wandler-Baueinheit (2), und eine zweite Schnittstelle (52), verbunden mit dem Ultraschallabbildungs-Wandler (31), umfasst,
wobei jeder von den Ultraschallbehandlungs-Wandlern (21) ein nicht geschlossener umlaufender Wafer ist und ein Umfangswinkel von dem nicht geschlossenen umlaufenden Wafer größer als 0 Grad und weniger als 360 Grad ist,
wobei Spalten ohne Übertragen akustischer Abstrahlungen von mindestens zwei nicht geschlossenen umlaufenden Wandlern ausgelegt sind, um parallel mit oder abgestuft miteinander zu sein,
wobei eine Achse von jedem von den nicht geschlossenen umlaufenden Wafern mit der Achse des Katheterkörpers (1) übereinstimmen,
**dadurch gekennzeichnet, dass**
der Ultraschallablations-Neuromodulationskatheter weiter mindestens einen unabhängigen Ultraschallabbildungs-Wandler (7) umfasst, der von einer Streifenform ist und sich an dem Spalt von dem nicht geschlossenen umlaufenden Wafer befindet.

2. Ultraschallablations-Neuromodulationskatheter nach Anspruch 1, **dadurch gekennzeichnet, dass** jeder von den Ultraschallbehandlungs-Wandlern (21) ein unter Röntgenstrahlen sichtbarer piezo-elektrischer Wafer ist und ein unter Röntgenstrahlen sichtbarer Beschichtungsfilm auf eine Oberfläche von dem piezo-elektrischen Wafer beschichtet ist.

3. Ultraschallablations-Neuromodulationskatheter nach Anspruch 2, **dadurch gekennzeichnet, dass** der Ultraschallabbildungs-Wandler (31) einen streifenförmigen Wafer mit einer bogenförmigen Oberfläche oder ein umlaufender Wafer ist und die akustischen Abstrahlungen, die von jedem von den Ultraschallbehandlungs-Wandlern (21) emittiert werden, in einer bildgebenden Fläche von dem benachbarten Ultraschallabbildungs-Wandler (31) vorliegen.

4. Ultraschallablations-Neuromodulationskatheter nach Anspruch 3, **dadurch gekennzeichnet, dass** der Ultraschallabbildungs-Wandler (31) der umlaufende Wafer ist und der umlaufende Wafer von dem Ultraschallabbildungs-Wandler (31) eine Achse aufweist, die mit einer Achse von dem nicht geschlossenen umlaufenden Wafer von jedem von den Ultraschallbehandlungs-Wandlern (21) übereinstimmt.

5. Ultraschallablations-Neuromodulationskatheter nach Anspruch 4, **dadurch gekennzeichnet, dass** der Steuergriff (4) zum Steuern des Katheterkörpers (1) zum unidirektionalen Rotieren ausgelegt ist;
ein Federstück und ein keilförmiger Antrieb in dem Steuergriff (4) bereitgestellt werden, und der Katheterkörper (1) mit dem Steuergriff (4) über den keilförmigen Antrieb drehbar verbunden ist; und
das Federstück ein Ende aufweist, befestigt an dem Steuergriff (4), und anderes Ende, das gegen den keilförmigen Antrieb presst, zum Verhindern, dass der keilförmige Antrieb umgekehrt rotiert.

6. Ultraschallablations-Neuromodulationskatheter nach Anspruch 5, **dadurch gekennzeichnet, dass** ein Stellknopf (8) zum Bestimmen eines Drehwinkels des Katheterkörpers (1) auf einer umlaufenden Oberfläche des Steuergriffs (4) bereitgestellt wird.

7. Ultraschallablations-Neuromodulationskatheter nach Anspruch 6, **dadurch gekennzeichnet, dass** weiter mindestens zwei unter Röntgenstrahlen sich sichtbar entwickelnde Marker (6) enthalten sind, wobei
die sich entwickelnden Marker (6) ausgelegt sind, den Bedienenden beim Gewinnen einer Position des Ultraschallablations-Katheters beim Eintreten in einen menschlichen Körperhohlraum und des Drehwinkels des Ultraschallablations-Katheters zu unterstützen;
mindestens zwei sich entwickelnde Marker (6) getrennt an dem Katheterkörper (1) an Positionen an zwei Enden des Ultraschallbehandlungs-Wandlers (21) angeordnet sind; und
jeder von den sich entwickelnden Markern (6), die auf dem Katheterkörper (1) angeordnet sind, Blatt-förmig ist.

## Revendications

1. Cathéter de neuromodulation d'ablation ultrasonore, comprenant un corps de cathéter (1), un ensemble de transducteur de traitement ultrasonore (2), un ensemble de transducteur d'imagerie ultrasonore (3), une poignée de commande (4) et une interface de transducteur (5), dans lequel :
le corps de cathéter (1) est de structure cylindrique ;
l'ensemble de transducteur de traitement ultrasonore (2) est agencé sur une surface latérale d'une extrémité distante du corps de cathéter (1), l'ensemble de transducteur de traitement ultrasonore (2) comprend au moins deux transducteurs de traitement ultrasonores (21), et une distance entre deux transducteurs de traitement ultrasonores (21) adjacents est supérieure ou égale à 1 cm ;
l'ensemble de transducteur d'imagerie ultrasonore (3) comprend au moins un transducteur d'imagerie ultrasonore (31), et le transducteur d'imagerie ultrasonore (31) est agencé entre deux transducteurs de traitement ultrasonores (21) adjacents ;
la poignée de commande (4) est agencée à une extrémité proche du corps de cathéter (1) ; et
l'interface de transducteur (5) comprend une première interface (51) reliée à l'ensemble de transducteur de traitement ultrasonore (2) et une seconde interface (52) reliée au transducteur d'imagerie ultrasonore (31),
dans lequel chacun des transducteurs de traitement ultrasonores (21) est une tranche circonférentielle non fermée et un angle circonférentiel de la tranche circonférentielle non fermée est supérieur à 0 degré et inférieur à 360 degrés,
dans lequel les intervalles sans faisceaux acoustiques de transmission d'au moins deux transducteurs circonférentiels non fermés sont configurés pour être parallèles ou décalés les uns par rapport aux autres,
dans lequel un axe de chacune des tranches circonférentielles non fermées coïncide avec l'axe du corps de cathéter (1),
**caractérisé en ce que**
le cathéter de neuromodulation d'ablation ultrasonore comprend en outre au moins un transducteur d'imagerie ultrasonore (7) indépendant, qui est en forme de bande et est situé au niveau de l'intervalle de la tranche circonférentielle non fermée.

2. Cathéter de neuromodulation d'ablation ultrasonore selon la revendication 1, **caractérisé en ce que** chacun des transducteurs de traitement ultrasonores (21) est une tranche piézoélectrique visible aux rayons X et un film de revêtement visible aux rayons X est appliqué sur une surface de la tranche piézoélectrique.

3. Cathéter de neuromodulation d'ablation ultrasonore selon la revendication 2, **caractérisé en ce que** le transducteur d'imagerie ultrasonore (31) est une tranche en forme de bande avec une surface incurvée ou une tranche circonférentielle, et les faisceaux acoustiques émis par chacun des transducteurs de traitement ultrasonores (21) sont dans une zone d'imagerie du transducteur d'imagerie ultrasonore (31) adjacent.

4. Cathéter de neuromodulation d'ablation ultrasonore selon la revendication 3, **caractérisé en ce que**
le transducteur d'imagerie ultrasonore (31) est la tranche circonférentielle, et la tranche circonférentielle du transducteur d'imagerie ultrasonore (31) possède un axe coïncidant avec un axe de la tranche circonférentielle non fermée de chacun des transducteurs de traitement ultrasonores (21).

5. Cathéter de neuromodulation d'ablation ultrasonore selon la revendication 4, **caractérisé en ce que** la poignée de commande (4) est configurée pour commander le corps de cathéter (1) pour le faire tourner de manière unidirectionnelle ;
une pièce à ressort et un engrenage en forme de biseau sont prévus dans la poignée de commande (4) et le corps de cathéter (1) est relié de manière rotative à la poignée de commande (4) par l'intermédiaire de l'engrenage en forme de biseau ; et
la pièce à ressort a une extrémité fixée à la poignée de commande (4) et une autre extrémité appuyant contre l'engrenage en forme de biseau afin d'empêcher l'engrenage en forme de biseau de tourner dans le sens inverse.

6. Cathéter de neuromodulation d'ablation ultrasonore selon la revendication 5, **caractérisé en ce qu'**un cadran (8) pour déterminer un angle de rotation du corps de cathéter (1) est prévu sur une surface circonférentielle de la poignée de commande (4).

7. Cathéter de neuromodulation d'ablation ultrasonore selon la revendication 6, **caractérisé en ce qu'**il comprend en outre au moins deux marqueurs de développement (6) visibles aux rayons X, dans lequel
les marqueurs de développement (6) sont configurés pour aider l'opérateur à obtenir une position du cathéter d'ablation ultrasonore entrant dans une cavité du corps humain et l'angle de rotation du cathéter d'ablation ultrasonore ;
au moins deux marqueurs de développement (6) sont situés séparément au niveau du corps de cathéter (1) à des positions situées à deux extrémités des transducteurs de traitement ultrasonores (21) ; et
chacun des marqueurs de développement (6) agencés sur le corps de cathéter (1) est en forme de feuille.
